# EUROPÄISCHE PATENTSCHRIFT

(11) **EP 1 009 450 B1**
(45) Veröffentlichungstag und Bekanntmachung des Hinweises auf die Patenterteilung: **03.04.2002**
(21) Anmeldenummer: 98951339.5
(22) Anmeldetag: 01.09.1998
(51) Int. Cl.: A61L 27/00

(54) **FALTBARE INTRAOKULARLINSE**
FOLDABLE INTRA-OCULAR LENS
CRISTALLIN ARTIFICIEL PLIABLE

(30) Priorität: 02.09.1997 DE 19738345
(43) Veröffentlichungstag der Anmeldung: 21.06.2000
(73) Patentinhaber: Müller-Lierheim, Wolfgang G.K., Dr., 81477 München (DE)
(72) Erfinder: Müller-Lierheim, Wolfgang G.K., Dr., 81477 München (DE)
(74) Vertreter: Nöth, Heinz, Dipl.-Phys.
(86) Internationale Anmeldenummer: EP9805540
(87) Internationale Veröffentlichungsnummer: WO9911303

(56) Entgegenhaltungen:
- EP-A- 0 492 126
- FR-A- 2 609 425
- FR-A- 2 757 065
- US-A- 4 304 895
- US-A- 4 731 079
- US-A- 5 002 570

## Beschreibung

Die Erfindung betrifft eine Intraokularlinse nach dem Oberbegriff des Patentanspruches 1.

Eine derartige Intraokularlinse ist aus der US 5,217,491 bekannt. Die bekannte Intraokularlinse besteht aus einem faltbaren optischen Linsenteil, aus einem weichen, insbesondere quellbaren Material, beispielsweise pHEMA (Polyhydroxyethylmethacrylat) oder einem Copolymer davon und einem härteren Haptikteil aus PMMA (Polymethylmethacrylat). In dieser Druckschrift ist ferner beschrieben, daß der Versuch unternommen wurde, einen Linsenrohling herzustellen, bei welchem der den optischen Linsenteil bildende Kern aus einem HEMA-MMA-Copolymer und ein diesen umgebender härterer Ring für die spätere Haptik aus PMMA gebildet sind. Bei einem pHEMA/MMA-Verhältnis von 75/25 konnte aus einem scheibenförmigen Rohling der Kern herausgedrückt werden. Bei einem pHEMA/MMA-Verhältnis von 50/50 war der Kern fest im äußeren Ring, jedoch löste sich der Kern nach Hydratisierung in Wasser aus dem PMMA-Ring.

Bei einem weiteren Versuch wurde ein Linsenrohling hergestellt, bei welchem der für den optischen Linsenteil bestimmte Kern aus einem HEMA-MMA-Copolymer mit einem HEMA/MMA-Verhältnis von 75/25 mit einem härteren Haptikring aus PMMA mit geringem HEMA-.Anteilen in einem Verhältnis von MMA/HEMA in der Ausgangslösung von 90/10 und 80/20 hergestellt wurde. Für den Linsenrohling mit einem pHEMA/MMA-Verhältnis von 75/25 ergab sich eine feste Bindung zwischen dem Haptikring und dem optischen Kern. Letzterer Linsenrohling hat jedoch keine ausreichende Langzeit-Hydrolysestabilität. Dies ist auch der Fall bei dem Linsenrohling, bei welchem der Kern durch Polymerisation von HEMA und TEGDMA als Vernetzer hergestellt ist, der Kern anschließend gequollen wird und dann um den Kern unter Verwendung von EGDMA als Vernetzer ein PMMA-Ring gebildet wird.

Aus der EP 0 269 288 A1 ist es bekannt, als Material für eine Intraoularlinse ein Copolymerisat von 80g HEMA und 20g MMA, zu verwenden. Femer ist es aus dieser Entgegenhaltung bekannt, einem Copolymerisat mit einem HEMA/MMA-Mischungsverhältnis von 65/35 EDGMA zuzusetzen.

Aus der DE 38 00 529 A1 ist für die Hersteiltung einer Intraokularlinse ein Copolymer aus 85% HEMA und 15% MMA, welche mit 0,5% EDGMA vernetzt sind, bekannt. Es handelt sich hier um gering vernetzte Copolymere.

Aus der US 4 871 785 sind in erster Linie zur Herstellung von Kontaktlinsen HEMA enthaltende Copolymerisate, welche auch für Intraokularlinsen geeignet sein sollen, bekannt, bei denen ein hoher Anteil an Vernetzungsmittel eingesetzt wird.

Insbesondere im biologischen Milieu des Auges erhält man keine ausreichende Langzeit-Hydrolyse-Stabilität. Dies liegt vor allem daran, daß das Kammerwasser und darin vorhandene Enzyme hydrolisierend auf das Linsenmaterial, insbesondere des optischen Teils, wirken.

Aufgabe der Erfindung ist es, eine Intraokularlinse der eingangs genannten Art zu schaffen, welche eine ausreichende Langzeit-Hydrolyse-Stabilität, insbesondere im biologischen Milieu des Auges, aufweist.

Diese Aufgabe wird erfindungsgemäß durch das Kennzeichen des Patentanspruches 1 gelöst.

Die Erfindung zeichnet sich dadurch aus, daß ein HEMA-MMA-Copolymer den optischen Linsenteil aus einer stark vernetzten Matrix mit dreidimensionalem Netzwerk bildet. Durch die starke Vernetzung und den MMA-Anteil wird die erforderliche Hydrolyse-Stabilität des den optischen Linsenteil bildenden Copolymers erreicht. Vor allem im biologischen Milieu des Auges erzielt man mit der erfindungsgemäßen Linse eine sehr gute LangzeitStabilität und Biokompatibilität. Von Vorteil ist ferner, daß die Intraokularlinse im Autoklaven bei 121°C dampfsterilisiert werden kann. Dabei besteht die Linse aus einem hydratisierbaren optischen Linsenteil und einer harten Haptik. In dieser Konsistenz kanri die Linse für die Implantation gefaltet werden, wobei die Haptik in bekannter Weise ausreichend steif ausgebildet sein kann, um eine exakte Positionierung der Intraokularlinse im implantierten Zustand im Auge, insbesondere in der Hinterkammer, zu gewährleisten.

Die Linse kann aus einem Kombinationsrohling hergestellt werden, dessen Kern von dem HEMA-MMA-Copolymer gebildet ist und welcher von einem harten Ring aus PMMA oder PEMA (Polyethylmethacrylat) umgeben ist. Aus dem harten Ring kann dann durch mechanische Bearbeitung, beispielsweise Schleifen oder dergl., die endgültige Haptikform hergestellt werden.

Zur Erzielung der starken Vernetzung kommt bei einem Kombinationsrohling ein gemeinsamer Vernetzer für das HEMA-MMA-Copolymer des optischen Linsenteils und das PMMA oder PEMA des Haptikteils zum Einsatz. Dieser Vernetzer besitzt wenigstens zwei funktionelle Reaktionsgruppen, welche mit völlig unabhängig voneinander vorliegenden Ketten der Ausgangsmonomere reagieren. Auf diese Weise werden die Ketten zu einem dreidimensionalen Netzwerk verbunden. Als Vernetzer kommt Ethylenglykoldimethacrylat (EGDMA) zum Einsatz. Der Anteil an HEMA im Copolymer des optischen Linsenteils kann 80 bis 95 Gew.-% betragen. Der Anteil an MMA im Copolymer beträgt etwa 4 bis 17 Gew.-%. Der Anteil an Vernetzer im Copolymer beträgt etwa 0,5 bis 2,0 Gew.-%.

Die Herstellung des Linsenrohlings kann auf folgende Weise erfolgen: In eine Polymerisationsform, bevorzugterweise aus Polypropylen, wird ein gelochtes Scheibchen aus PMMA oder PEMA mit Preßsitz eingepaßt. Diese aus der Polymerisationsform und dem gelochten Scheibchen bestehende Kombination wird in die Polymerisationsanlage überführt. In die Mittenbohrung des Scheibchens wird die zu polymerisierende Lösung, welche HEMA, MMA, den Vemetzer, insbesondere EGDMA, und einen UV-Absorber enthält, eingebracht und anschließend polymerisiert.

Die Polymerisation erfolgt in herkömmlicher Weise und beruht auf radikalischer Polymerisation. Der UV-Absorber ist insbesondere 4-Methacryloxy-2-hydroxybenzophenon (MAO-2-HBP) mit der Formel

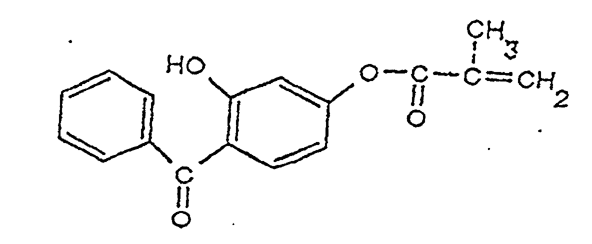

Dieser UV-Absorber enthält ein Reaktionszentrum (=CH₂), welches den Einbau in die dreidimensionale Polymermatrix ermöglicht.

Zur Initiierung der Polymerisation dient ein geeigneter Starter, insbesondere chemischer Starter, beispielsweise α,α'-Azoisobutyronitril (AIBN). Die Startersubstanz wird in einem Anteil von etwa 0,1 Gew.-% zugegeben. Die Reaktionsmischung wird in der Form bei erhöhter Temperatur, insbesondere bei 40°C, etwa 48 Stunden durchpolymerisiert. Anschließend erfolgt eine Nachtemperung des Polymerisats bei etwa 60°C bis 70°C für ca. fünf Stunden. Gegebenenfalls kann eine Nachbehandlung in einem Vakuumtrockenschrank bei Temperaturen zwischen 70° und 100°C, z.B. bei 50 mbar, erfolgen. Das fertige Copolymerisat bildet den Kern, der ringförmig vom gelochten Scheibchenmaterial umfaßt ist. Im Scheibchenmaterial für den Haptikteil kann ferner ein Farbstoff, z.B. Solvent Green 3, gelöst sein. In einem geringen Anteil kann auch HEMA zugesetzt werden. Hierdurch wird erreicht, daß die Haptik weniger spröde wird. Eine Haptik mit geringer Sprödigkeit wird auch durch PEMA erreicht.

Die beschriebene Vorgehensweise resultiert in einer festen Verbindung zwischen dem Kern aus dem HEMA-MMA-Copolymer und dem diesen Kern umgebenden Ring aus PMMA oder PEMA. Durch die Polymerisation des Kerns innerhalb des Ringes bzw. gelochten Scheibchens, aus dem die Haptik für die Intraokularlinse, beispielsweise durch mechanische Bearbeitung, gewonnen werden kann, erreicht man eine hervorragende Verbindung zwischen dem hydratisierbaren Kern aus dem HEMA-MMA- Copolymer, welches den optischen Linsenteil der Intraokularlinse bildet, und dem Ring aus PMMA oder PEMA. Das hydratisierte Copolymer, welches eine hohe Hydrolyse-Stabilität aufweist, ist für die Implantation gut faltbar.

Aufgrund der starken Vernetzung auch des Haptikteilmaterials kann die fertige Intraokularlinse in hydratisiertem Zustand der Linsenoptik mit Dampfsterilisation behandelt werden. Dies hat als günstigen Nebeneffekt die Freiheit von Rückständen und Abbauprodukten und macht die parametrische Freigabe ohne Entgasung nach der Ethylenoxyd(EO)-Sterilisation oder Wärmebehandlung nach der Strahlensterilisation möglich.

Die stabile Verbindung zwischen dem optischen Linsenteil und der Haptik ergibt sich auch aufgrund der chemischen Verwandtschaft des HEMA-MMA-Copolymers mit dem PMMA oder PEMA und die Verwendung des gemeinsamen Vemetzers EGDMA.

Im folgenden sind Ausführungsbeispiele für Zusammensetzungen des optischen Linsenteils angegeben.

### Optikteil Gew.-%

| | | Gew.-% |
|---|---|---|
| 1. | HEMA | 89,70 |
| | MMA | 8,00 |
| | EGDMA: | 2,00 |
| | MAO-2-HBP: | 0,25 |
| | AIBN: | 0,05 |

| | | Gew.-% |
|---|---|---|
| 2. | HEMA: | 82,00 |
| | MMA | 16,00 |
| | EGDMA: | 0,50 |
| | MAO-2-HBP: | 1,00 |
| | AIBN: | 0,50 oder weniger |

## Patentansprüche

1. Intraokularlinse mit einem faltbaren optischen Linsenteil aus einem HEMA-MMA-Copolymer und einem härteren Haptikteil, **dadurch gekennzeichnet, daß** das HEMA-MMA-Copolymer des optischen Linsenteils und der Haptikteil, welcher aus PMMA oder PEMA besteht, durch einen gemeinsamen Vernetzer, der EGDMA ist, stark zu einem dreidimensionalen Netzwerk vernetzt sind und daß der Anteil von HEMA im Copolymer des optischen Linsenteils mindestens 80 Gew.-% und der Anteil an MMA etwa 4 bis 17 Gew.-% betragen.

2. Intraokularlinse nach Anspruch 1, **dadurch gekennzeichnet, daß** der Anteil von HEMA im Copolymer des optischen Linsenteils etwa bis 95 Gew.-% beträgt.

3. Intraokularlinse nach Anspruch 1 oder 2, **dadurch gekennzeichnet, daß** ein UV-Absorber, welcher wenigstens eine funktionelle Reaktionsgruppe, insbesondere =CH₂ aufweist, in die Copolymermatrix des optischen Linsenteils einpolymerisiert ist.

4. Intraokularlinse nach Anspruch 3, **dadurch gekennzeichnet, daß** der UV-Absorber 4-Methacryloxy-2-hydroxybenzophenon ist.

5. Intraokularlinse nach einem der Ansprüche 1 bis 4, **gekennzeichnet durch** die Herstellung aus einem Rohling, bei dem in einem PMMA- oder PEMA- Ring das Copolymer für den optischen Linsenteil polymerisiert ist.

6. Intraokularlinse nach Anspruch 5, **dadurch gekennzeichnet, daß** vor der Polymerisation des Copolymers für den optischen Linsenteil der PMMA- oder PEMA- Ring vorgequollen ist.

7. Intraokularlinse nach Anspruch 5 oder 6, **dadurch gekennzeichnet, daß** vor der Polymerisation des Copolymers des optischen Linsenteils MMA aus der zu polymerisierenden Copolymer-Lösung für den optischen Linsenteil in den PMMA-Ring eindiffundiert ist.

8. Intraokularlinse nach einem der Ansprüche 1 bis 7, **dadurch gekennzeichnet, daß** die Linse dampfsterilisiert ist.

## Claims

1. An intra-ocular lens comprising a foldable optical lens portion comprising an HEMA-MMA copolymer and a harder haptic portion, **characterised in that** the HEMA-MMA copolymer of the optical lens portion and the haptic portion which comprises PMMA or PEMA are strongly crosslinked to form a three-dimensional network by a common cross-linking agent which is EGDMA, and that the proportion of HEMA in the copolymer of the optical lens portion is at least 80% by weight and the proportion of MMA is about 4 to 17% by weight.

2. An intra-ocular lens according to claim 1 **characterised in that** the proportion of HEMA in the copolymer of the optical lens portion is up to about 95% by weight.

3. An intra-ocular lens according to claim 1 or claim 2 **characterised in that** a UV-absorber which has at least one functional reaction group, in particular =CH₂ is polymerised into the copolymer matrix of the optical lens portion.

4. An intra-ocular lens according to claim 3 **characterised in that** the UV-absorber is 4-methacryloxy-2-hydroxybenzophenone.

5. An intra-ocular lens according to one of claims 1 to 4 **characterised by** production from a blank in which the copolymer for the optical lens portion is polymerised in a PMMA- or PEMA-ring.

6. An intra-ocular lens according to claim 5 **characterised in that** the PMMA- or PEMA-ring is pre-swelled prior to polymerisation of the copolymer for the optical lens portion.

7. An intra-ocular lens according to claim 5 or claim 6 **characterised in that** prior to polymerisation of the copolymer of the optical lens portion MMA is diffused into the PMMA-ring, out of the copolymer solution to be polymerised, for the optical lens portion.

8. An intra-ocular lens according to one of claims 1 to 7 **characterised in that** the lens is steam-sterilised.

## Revendications

1. Lentille intra-oculaire avec une partie lentille optique pliable formée d'un copolymère de HEMA-MMA et une partie haptique plus dure, **caractérisée en ce que** le copolymère de HEMA-MMA de la partie lentille optique et la partie haptique, qui est formée de PMMA ou de PEMA, sont fortement réticulés en un réseau tridimensionnel au moyen d'un agent commun de réticulation qui est du EGDMA et **en ce que** la proportion de HEMA dans le copolymère de la partie lentille optique est d'au moins 80 % en poids et la proportion de MMA est d'environ 4 à 17 % en poids.

2. Lentille intra-oculaire selon la revendication 1, **caractérisée en ce que** la proportion de HEMA dans le copolymère de la partie lentille est d'environ 95 % en poids.

3. Lentille intra-oculaire selon la revendication 1 ou 2, **caractérisée en ce qu'**un absorbant de rayons ultraviolets qui présente au moins un groupe fonctionnel de réaction, en particulier =CH₂, est inséré par polymérisation dans la matrice copolymère de la partie lentille optique.

4. Lentille intra-oculaire selon la revendication 3, **caractérisée en ce que** l'absorbant de rayons ultraviolets est de la 4-méthacryloxy-2-hydroxybenzophénone.

5. Lentille intra-oculaire selon l'une des revendications 1 à 4, **caractérisée par** la fabrication à partir d'une ébauche dans laquelle le copolymère pour la partie lentille optique est polymérisé dans un anneau de PMMA ou de PEMA.

6. Lentille intra-oculaire selon la revendication 5, **caractérisée en ce que** l'anneau de PMMA ou de PEMA est préalablement gonflé avant la polymérisation du copolymère pour la partie lentille optique.

7. Lentille intra-oculaire selon la revendication 5 ou 6, **caractérisée en ce qu'**on fait diffuser du MMA provenant de la solution de copolymère à polymériser pour la partie lentille optique dans l'anneau de PMMA avant la polymérisation du copolymère de la partie lentille optique.

8. Lentille intra-oculaire selon l'une des revendications 1 à 7, **caractérisée en ce que** la lentille est stérilisée à la vapeur.
